**Europäisches Patentamt**

(19) **European Patent Office**

**Office européen des brevets**

(11) Veröffentlichungsnummer: **0 004 653**
**B1**

(12) # EUROPÄISCHE PATENTSCHRIFT

(45) Veröffentlichungstag der Patentschrift:
**15.07.81**

(51) Int. Cl.³: **C 07 C 27/06,** C 07 C 31/08,
C 07 C 47/06, C 07 C 53/08

(21) Anmeldenummer: **79100992.1**

(22) Anmeldetag: **31.03.79**

(54) Verfahren zur Herstellung sauerstoffhaltiger Kohlenstoffverbindungen aus Synthesegas.

(30) Priorität: 04.04.78 DE 2814365
10.06.78 DE 2825495
18.11.78 DE 2850110

(43) Veröffentlichungstag der Anmeldung:
**17.10.79 Patentblatt 79/21**

(45) Bekanntmachung des Hinweises auf die Patenterteilung:
**15.07.81 Patentblatt 81/28**

(84) Benannte Vertragsstaaten:
**BE DE FR GB IT NL**

(56) Entgegenhaltungen:
**FR-A-2 277 804**
**FR-A-2 234 256**
**FR-A-2 234 257**
**FR-A-2 296 608**
**FR-A-2 296 609**
**FR-A-2 317 260**
**FR-A-2 326 397**
**FR-A-2 326 399**
**US-A-3 989 799**

(73) Patentinhaber: **HOECHST AKTIENGESELLSCHAFT,**
**Zentrale Patentabteilung Postfach 80 03 20,**
**D-6230 Frankfurt/Main 80 (DE)**

(72) Erfinder: **Schmidt, Hans-Joachim, Dr., Burgenblick 6,**
**D-6240 Königstein/Taunus (DE)**
Erfinder: **Wunder, Friedrich, Dr., Jahnstrasse 46,**
**D-6093 Flörsheim am Main (DE)**
Erfinder: **Arpe, Hans-Jürgen, Dr., Am Hirtengraben 19,**
**D-6233 Kelkheim (Taunus) (DE)**
Erfinder: **Leupold, Ernst Ingo, Dr., Am Zäunefeld 15,**
**D-6392 Neu-Anspach (DE)**

### Verfahren zur Herstellung sauerstoffhaltiger Kohlenstoffverbindungen aus Synthesegas

Die Erfindung betrifft ein Verfahren zur Herstellung sauerstoffhaltiger Verbindungen mit vorzugsweise zwei Kohlenstoffatomen im Molekül und insbesondere die Herstellung von Essigsäure, Ethanol, Acetaldehyd und gegebenenfalls ihren Folgeprodukten durch Umsetzung von Synthesegas, d.h. Kohlenmonoxid und Wasserstoff, an Rhodium-Katalysatoren.

Die Bildung sauerstoffhaltiger Verbindungen mit zwei oder mehr Kohlenstoffatomen im Molekül bei Umsetzungen von Synthesegas an Katalysatoren unterschiedlicher Zusammensetzung ist bereits aus zahlreichen Veröffentlichungen und Verfahren bekannt. Diese Verbindungen werden jedoch im allgemeinen nur als Nebenprodukte oder in wenig spezifischer, breiter Produktverteilung, d.h. mit nur geringen Konzentrationen der einzelnen Komponenten erhalten, so dass solche Verfahren für eine wirtschaftliche Herstellung der gewünschten Produkte nicht in Betracht kommen.

Aus FR-A-2 296 608, FR-A-2 296 609, FR-A-2 326 399, FR-A-2 326 397, FR-A-2 234 257, FR-A-2 277 804, FR-A-2 234 256 und US-A-3 989 799 ist die Herstellung von Polyalkoholen bzw. Methanol aus Synthesegas an Rhodiumcarbonyl-Katalysatoren bekannt.

Aus den deutschen Auslegeschriften 2 503 233 und 2 503 204 sowie FR-A-2 317 260 ist weiter bekannt, dass man im Gegensatz zu sonstigen Katalysatorsystemen die Gasphasenumsetzung von Synthesegas an Rhodiummetall enthaltenden Katalysatoren im wesentlichen zu Gemischen sauerstoffhaltiger Produkte mit zwei Kohlenstoffatomen im Molekül, wie Essigsäure, Ethanol und/oder Acetaldehyd, führen kann. Die Selektivität zu den einzelnen Verbindungen hängt von den Reaktionsbedingungen und der Katalysatorzusammensetzung ab und kann durch Zusatz von Eisensalzen zugunsten von Ethanol beeinflusst werden. Weiter ist aus der deutschen Offenlegungsschrift 2 628 463 auch bekannt, dass Mangan als Cokatalysator wirken und die Aktivität der Rhodiummetallkatalysatoren verbessern kann. In FR-A-2 317 260 wird angegeben, dass Magnesiumoxid als Träger für Rhodiummetall dienen kann, jedoch dürfte es kaum gegen die bei der Umsetzung entstehende Essigsäure beständig sein.

Die zuletzt genannten Verfahren basieren auf der Verwendung von Rhodiummetall als katalytisch wirksamer Komponente. Bei der Herstellung der Katalysatoren werden deshalb Bedingungen gewählt, die eine praktisch quantitative Bildung metallischen Rhodiums gewährleisten. Hierzu gehören die Reduktion von Rhodiumchlorid oder Rhodium-dicarbonylacetylacetonat im Wasserstoffstrom bei Temperaturen oberhalb 300 °C, vorzugsweise bei Temperaturen von 450–500 °C, sowie – im Fall der Verwendung von Rhodiumnitrat – die Anwendung einer zusätzlichen Pyrolysestufe mit anschliessender Reduktion.

Bei der Umsetzung von Synthesegas an Rhodiummetallkatalysatoren können unter bestimmten Versuchsbedingungen zwar hohe Aktivitäten und Raumzeitausbeuten von über 400 g sauerstoffhaltigen $C_2$-Produkten pro Liter Katalysator und Stunde erzielt werden, die Selektivität zu den gewünschten Produkten ist aber noch unbefriedigend. So wird z.B. nach Tabelle 2 der deutschen Auslegeschrift 2 503 233 mit einem Katalysator, der 5 Gew.-% Rhodiummetall auf Kieselsäure enthält, ein Reaktionsprodukt erhalten, das 42,2 Gew.-% Essigsäure, Ethanol und Acetaldehyd enthält, entsprechend einer Selektivität von 40,2% bezogen auf umgesetztes Kohlenmonoxid. Das restliche Kohlenmonoxid wird im wesentlichen zu Methan und Kohlendioxid umgesetzt.

Somit ergibt sich die Aufgabe, Rhodium enthaltende Katalysatoren zur Umsetzung von Synthesegas in ihrer Selektivität zu sauerstoffhaltigen $C_2$-Produkten zu verbessern, um damit die Wirtschaftlichkeit eines solchen Herstellverfahrens für diese technisch bedeutenden Zwischenprodukte zu erhöhen.

Es wurde nun gefunden, dass Aktivität und/oder Selektivität der Rhodiumkatalysatoren entscheidend von den Reduktionsbedingungen abhängen, die bei der Katalysatorherstellung angewendet werden, und dass bei Einhaltung niederer Reduktionstemperaturen Katalysatoren mit verbesserter Selektivität und/oder Aktivität erhalten werden. Als Ursache für diese Eigenschaften wurde erkannt, dass in diesen Katalysatoren im Gegensatz zu der literaturbekannten Verwendung von Rhodium in metallischer Form hier das Rhodium nicht als Metall, sondern als Salz oder Komplexverbindung des Rhodiums in einer Wertigkeitsstufe unter drei vorliegt, also z.B. als ein- oder zweiwertiges Rhodium oder auch als Gemisch dieser niederen Wertigkeitsstufen. Neben dem Rhodium müssen Halogenidionen, gegebenenfalls im Gemisch mit anderen Anionen, anwesend sein. Ausserdem wurde gefunden, dass Aktivität und/oder Selektivität dieser Katalysatoren durch Zusatz von Salzen oder Verbindungen des Magnesiums als Cokatalysator wesentlich verbessert werden.

Gegenstand der Erfindung ist daher ein Verfahren zur Herstellung von Essigsäure, Ethanol, Acetaldehyd und gegebenenfalls ihren Folgeprodukten durch Umsetzung von Kohlenmonoxid mit Wasserstoff in Gegenwart von Rhodium, das dadurch gekennzeichnet ist, dass man einen Katalysator verwendet, der auf einem Träger Salze oder Komplexverbindungen des Rhodiums in einer Wertigkeitsstufe unter drei, Halogenidionen und als Cokatalysator Magnesium in Form von Salzen oder Komplexverbindungen enthält.

Der Befund, dass Rhodium in einer niederen Wertigkeitsstufe die Synthesegasumsetzung zu sauerstoffhaltigen $C_2$-Produkten besonders vorteilhaft katalysiert und unter den Reduktions- bzw. Reaktionsbedingungen im wesentlichen nicht zum Metall reduziert wird, war überraschend und nicht vorauszusehen, da bekannt ist, dass schon unter relativ milden Bedingungen, beispielsweise unter 100°C, Rhodiumsalze bei der Behandlung mit Wasserstoff quantitativ zu Rhodiummetall reduziert werden (vgl. Newkirk et al., J. of Catal. 11 (1968), 370). Es hat sich jedoch herausgestellt, dass in Gegenwart von Magnesiumionen als Cokatalysator und von Halogenidionen die Reduktion von auf Trägern aufgebrachten Rhodiumsalzen oder Rhodium-Komplexverbindungen mit Wasserstoff bei Temperaturen unter 30°C nicht zum Metall führt. Ferner zeigte sich, dass auch bei der Umsetzung des aus Kohlenmonoxid und Wasserstoff bestehenden Gasgemisches unter den Reaktionsbedingungen nach längerer Versuchszeit an dem Zustand des Rhodiums in dieser niederen (nichtmetallischen) Wertigkeitsstufe keine wesentliche Veränderung eintritt, wie sich aus dem Verhalten des Katalysators beim Start der Synthesegas-Umsetzung und nach längerer Reaktionszeit, sowie auch aus dem Verhältnis von Rhodium zu Halogen ergibt.

Sauerstoffhaltige Kohlenstoffverbindungen, die bei dem erfindungsgemässen Verfahren in hoher Selektivität entstehen, sind Essigsäure, Ethanol und/oder Acetaldehyd, ferner solche Produkte, die in einer Folgereaktion, z.B. durch Veresterung oder Kondensation, aus diesen Produkten unter den Reaktionsbedingungen gebildet werden können. Hierzu zählen u.a. Ethylacetat und das Diethylacetal des Acetaldehyds. Der Anteil an anderen sauerstoffhaltigen Verbindungen mit drei oder mehr Kohlenstoffatomen im Molekül ist sehr gering und liegt normalerweise unter 5 Mol-%, bezogen auf umgesetztes Kohlenmonoxid. Die Gesamtselektivität zu sauerstoffhaltigen $C_2$-Verbindungen, einschliesslich der in Ethylacetat und Acetaldehyddiethylacetal umgewandelten Produkte, beträgt bis zu 90%, bezogen auf umgesetztes Kohlenmonoxid. Das restliche Kohlenmonoxid wird, ausser zu den genannten Produkten mit 3 und mehr Kohlenstoffatomen, im wesentlichen zu Methan und anderen gasförmigen Kohlenwasserstoffen und im geringen Masse zu Kohlendioxid umgesetzt.

Zum Aufbau des Katalysators für das erfindungsgemässe Verfahren kann man von halogenhaltigen Salzen oder Komplexverbindungen des Rhodiums ausgehen. Geeignet sind Chloride, Bromide und/oder Jodide des Rhodiums oder auch Doppelsalze mit Alkalihalogeniden, wie z.B. Dikaliumtrichlororhodat. Geeignet sind ferner halogenhaltige Komplexverbindungen, die neben Rhodium und Halogen noch komplexbildende Liganden, wie Trialkylphosphine, Triarylphosphine, Ethylendiamin, Pyridin, Kohlenmonoxid, Olefine oder Wasser enthalten, also z.B. Tris-triphenylphosphin-rhodium-I-chlorid, -bromid oder-jodid, Tris-triphenylphosphin-rhodium-III-chlorid, Dichlor-bis-ethylendiamin-rhodium-I-chlorid, Tris-ethylendiamin-rhodium-III-chlorid, Bis-tri-o-tolyl-phosphin-rhodium-II-chlorid, Carbonyl-bistriphenyl-phosphin-rhodium-I-bromid oder Dicäsium-carbonyl-pentachloro-rhodat-III. Besonders geeignet sind Komplexe, die sowohl Rhodium als auch Magnesium enthalten, wie z.B. $Mg_3[Rh\ Cl_6]_2$, das man durch Umsetzung von Magnesiumchlorid mit Rhodiumchlorid in Essigsäure bei 100°C erhält, oder $Mg[Rh\ (CO)_2Cl_2]_2$, das aus Magnesiumchlorid und $[Rh\ (CO)_2Cl]_2$ in einem Gemisch aus Methanol, Chloroform und Chlorwasserstoff bei 20°C unter Stickstoff entsteht.

Darüber hinaus kommen auch solche Verbindungen des Rhodiums in Betracht, in denen es ionogen oder komplex an einen Träger gebunden ist. Beispiele hierfür sind die mit Rhodiumhalogeniden ausgetauschten Zeolithe und Ionenaustauscher, ferner komplex an Magnesiumsilikat gebundenes Rhodium. In diesen Verbindungen können neben Magnesium auch andere Metalle vorhanden sein, wie z.B. Lithium, Beryllium, Calcium, Mangan, Eisen, Kobalt oder Nickel.

Die eigentliche Herstellung des Katalysators kann nun in der Weise erfolgen, dass die katalytisch aktiven Salze oder Komplexverbindungen des ein- oder zweiwertigen Rhodiums als solche auf einen Träger aufgebracht werden, auf dem sie dann nach Imprägnierung eines Magnesiumsalzes schon ohne Reduktion wirksam sind. Man kann aber auch Salze oder Komplexverbindungen des Rhodiums verwenden, in denen es dreiwertig ist und anschliessend an die Imprägnierung durch geeignete Reduktion (wie weiter unten beschrieben) die katalytisch wirksamen Rhodiumsalze herstellen, in denen es in einer Wertigkeitsstufe unter drei (aber nicht als Metall) vorliegt. Diese gezielte Reduktion kann auch unter den Bedingungen der Synthesegasumsetzung, d.h. durch Reduktion mit Gemischen von Wasserstoff und Kohlenmonoxid, erfolgen.

Als Cokatalysatoren oder Aktivatoren werden erfindungsgemäss Salze oder Komplexverbindungen des Magnesiums verwendet. Hierfür eignen sich einfache anorganische und organische Salze des Magnesiums, wie z.B. das Chlorid, Bromid, Nitrat, Formiat, Acetat. Ferner können das Oxid, das Hydroxid, oder die Carbonate des Magnesiums verwendet werden, wenn man sie durch Behandlung mit Mineralsäuren oder Carbonsäuren in die genannten Salze überführt. Als Komplexverbindungen sind z.B. die oben erwähnten Magnesium-Rhodium-Komplexe besonders geeignet.

Die Magnesiumverbindungen können zusammen mit der Rhodiumverbindung auf einen Träger aufgebracht werden. Jedoch kann das cokatalytisch wirksame Magnesium auch vorher auf den Träger aufgebracht oder auch in eine Gerüstsubstanz eingebaut sein, beispielsweise in eine Silikat- oder Aluminiumoxid enthaltende Trägersubstanz wie Kieselsäure, Aluminiumoxid oder Aluminiumsilikat.

Eine weitere vorteilhafte Möglichkit besteht darin, Magnesium mittels Ionenaustausch an Kationenaustauscher zu binden, die auch als Träger für das Rhodium geeignet und unter den Versuchsbedingungen beständig sind, beispielsweise die als Molsiebe bekannten natürlichen oder synthetischen Aluminiumsilikate. Es führt aber auch die andere Reihenfolge der Trägerimprägnierung, d.h. zunächst die Imprägnierung mit Rhodiumverbindungen und dann mit Magnesiumverbindungen zu geeigneten Katalysatoren.

Das Halogenid kann als entsprechende Rhodium- und/oder als entsprechende Magnesiumverbindung aufgebracht werden; geeignete Verbindungen sind bereits erwähnt worden.

Man kann aber auch halogenfreie Rhodium- bzw. Magnesiumverbindungen, z.B. die Acetate oder Nitrate einsetzen und durch anschliessende Behandlung mit Halogenwasserstoff oder Imprägnierung mit einem Metallhalogenid die Halogenidionen auf den Träger aufbringen. Man kann auch mit einer Halogen enthaltenden organischen Verbindung (wie z.B. 1,1-Di-chloräthan), aus der Halogen freizusetzen ist, den für die selektive Synthesegasumsetzung erforderlichen Halogengehalt des Katalysators nach der Imprägnierung der Rhodium- und/oder Magnesiumverbindung einstellen.

Als Katalysatorträger können übliche Trägermaterialien mit unterschiedlichen spezifischen Oberflächen verwendet werden. Allerdings werden Träger mit spezifischen Oberflächen von 50 bis 1000 m²/g bevorzugt. Geeignet sind z.B. Kieselsäure, natürliche oder synthetische Silikate von Elementen der II. bis VIII. Gruppe des Periodischen Systems (also beispielsweise die Silikate des Magnesiums, Calciums, Aluminiums, der Seltenen Erde, des Titans, Zirkons, Mangans), ferner Aluminiumoxid, Titandioxid, Zirkondioxid, Thoriumdioxid, Zeolithe und Spinelle.

Zur Herstellung der Katalysatoren werden die Träger mit den aktiven Komponenten, wie zuvor angegeben, gleichzeitig oder in aufeinanderfolgenden Stufen getränkt oder imprägniert. Bei Einsatz von Rhodium-III-salzen ist die nachfolgende Behandlung mit geeigneten Reduktionsmitteln, wie Wasserstoff, Kohlenmonoxid, Methanol ein wesentlicher Teil der Katalysatorherstellung. Diese Reduktion kann in einer getrennten Apparatur oder im Reaktor selbst durchgeführt werden. Dabei werden solche Reduktionsbedingungen eingehalten, unter denen Rhodium in eine niedere (nichtmetallische) Wertigkeitsstufe überführt wird. Im allgemeinen sind hierfür Temperaturen unter 300°C, vorzugsweise zwischen 100 und 275°C, geeignet. Vielfach ist es zweckmässig, die Reduktion nicht mit den unverdünnten reduzierend wirkenden Gasen, sondern mit einem zusätzlichen Anteil an Inertgasen, wie z.B. Stickstoff, Kohlendioxid oder auch Edelgasen vorzunehmen.

Die Konzentration an Rhodium, Magnesium und Halogeniden in den Katalysatoren kann in weiten Grenzen variiert werden, im allgemeinen liegen die Werte zwischen 0,1 und 20 Gew.-% für Rhodium, zwischen 0,1 und 25 Gew.-% für Magnesium und zwischen 0,01 und 20 Gew.-% für die Halogenidionen. Bevorzugt sind Katalysatoren mit 1,0 bis 10 Gew.-% Rhodium, 0,1 bis 20 Gew.-% Magnesium und 0,05 bis 15 Gew.-% Halogeniden.

Zur Durchführung des erfindungsgemässen Verfahrens werden Gasgemische, die ganz oder zu einem überwiegenden Teil aus Kohlenmonoxid und Wasserstoff bestehen und daneben gegebenenfalls noch andere Komponenten wie Stickstoff, Argon, Kohlendioxid oder Methan enthalten können, über den Katalysator geleitet. Das molare Verhältnis von Kohlenmonoxid zu Wasserstoff kann dabei in weiten Grenzen variiert werden. Bevorzugt sind Molverhältnisse zwischen 5:1 und 1:5 und besonders zwischen 3:1 und 1:3. Die Reaktionstemperaturen liegen im allgemeinen zwischen 175 und 375°C, vorzugsweise zwischen 200 und 350°C, und die Reaktionsdrücke zwischen 1 und 300 bar, vorzugsweise zwischen 20 und 300 bar.

Zweckmässig ist es, Temperatur und Druck so aufeinander abzustimmen, dass eine hohe Selektivität zu den sauerstoffhaltigen Verbindungen gewährleistet ist und die bei höheren Temperaturen begünstigte exotherme Bildung von Methan gering gehalten wird. Man wird deshalb hohe Drücke und möglichst niedrige Temperaturen bevorzugen. Der Umsatz an Kohlenmonoxid sollte dabei im allgemeinen nicht über 50% liegen, da höhere Umsätze leicht zu vermehrter Nebenproduktbildung führen können, wobei neben Methan, Kohlendioxid und gasförmigen Kohlenwasserstoffen auch höhermolekulare flüssige Kohlenwasserstoffe und sauerstoffhaltige Produkte auftreten können.

Für die Verfahrensdurchführung ist die Gasphase bevorzugt. Hierzu können die herkömmlichen Festbettreaktoren verwendet werden, wobei es zur besseren Wärmeabführung vorteilhaft ist, die Katalysatorschichtdicke gering zu halten. Ferner sind auch Reaktoren mit bewegtem Katalysatorbett oder Wirbelbettreaktoren geeignet.

Man kann aber auch eine Umsetzung des Synthesegases in Gegenwart des festen und feinverteilten Katalysators, suspendiert in inerten Lösungsmitteln und/oder Reaktionsprodukten, durchführen.

Eine besonders bevorzugte Ausführungsform der Erfindung besteht darin, die Umsetzung in einer Kreisgasapparatur in der Gasphase durchzuführen, in der nach Abtrennung der kondensierbaren Reaktionsprodukte das nicht umgesetzte Gasgemisch wieder in den Reaktor zurückgeführt wird.

Diese Verfahrensweise ist besonders wirtschaftlich und ermöglicht durch Verdünnung des Frischgases mit dem im Kreislauf zurückgeführten wasserstoffärmeren Restgas höhere Reaktionstemperaturen und damit höhere Raumzeitausbeuten bei unveränderten Selektivitäten. Als Kreisgasapparaturen kommen dabei solche mit innerem oder äusserem Gasumlauf in Betracht.

Bei der Durchführung des erfindungsgemässen Verfahrens hat sich gezeigt, dass die Katalysatoren zwar eine hohe Anfangsaktivität und eine ausgezeichnete Selektivität der Kohlenmonoxidumsetzung zu den sauerstoffhaltigen $C_2$-Verbindungen aufweisen, dass aber bei längerem Einsatz der Katalysatoren, d.h. bei Betriebszeiten

von mehr als etwa 500 Stunden, Aktivität und Selektivität der Katalysatoren allmählich abfallen können. Die Katalysatoren haben infolgedessen teilweise nur eine begrenzte Lebensdauer.

Es wurde nun gefunden, dass man diese beträchtlich verlängern kann, wenn man während der Synthesegasumsetzung der Reaktionszone zusammen mit den gasförmigen Reaktionskomponenten Magnesiumsalze oder -verbindungen, die unter den Reaktionsbedingungen verdampfbar sind, kontinuierlich oder diskontinuierlich zuführt.

Diese bevorzugte Ausführungsform des erfindungsgemässen Verfahrens bietet den Vorteil, dass die für die Umsetzung verwendeten Katalysatoren in ihrer Aktivität und Selektivität auch nach mehr als 1000 Stunden noch nahezu unverändert sind.

Magnesiumsalze oder -verbindungen, die unter den Reaktionsbedingungen verdampfbar sind und dadurch gemeinsam mit einer oder mehreren der Reaktionskomponenten gasförmig in die Reaktionszone gebracht werden können, sind beispielsweise Magnesiumchlorid, -bromid, -jodid, -acetylacetonat, -ethylat, -isopropylat, Magnesiumaluminiummethylat und -isopropylat und die Magnesiumsalze aliphatischer Monocarbonsäuren mit 1-4 Kohlenstoffatomen im Molekül. Vorzugsweise verwendet man Magnesiumchlorid oder Magnesiumacetat. Es lassen sich aber auch solche Magnesiumsalze oder -verbindungen verwenden, die durch Umsetzung mit Halogenwasserstoff in das Halogenid oder durch Umsetzung mit aliphatischen Monocarbonsäuren in die entsprechenden Carboxylate übergeführt werden können, wie z.B. das Oxid, Hydroxid oder die Carbonate des Magnesiums.

Die flüchtigen Magnesiumsalze oder -verbindungen werden mit den gasförmigen Reaktionskomponenten in die Reaktionszone gebracht, wobei verschiedene Methoden angewendet werden können. So kann man die Magnesiumverbindung in gelöster Form, beispielsweise als Lösung in Wasser, Ethanol oder Essigsäure, in den heissen Gasstrom vor der Katalysatorschicht einspritzen. Es besteht weiter die Möglichkeit, die Reaktionsgase vor Eintritt in die Reaktonszone ganz oder teilweise bei erhöhter Temperatur mit einer Lösung oder Schmelze der Magnesiumverbindung in Berührung zu bringen oder diese Gase über eine solche Lösung bzw. Schmelze zu leiten. Eine besonders bevorzugte Ausführungsform besteht darin, die Reaktionskomponenten teilweise oder insgesamt über die in fester Form vorgelegte flüchtige Magnesiumverbindung bei erhöhter Temperatur zu leiten und so die Magnesiumverbindung ohne Verwendung eines zusätzlichen Lösungsmittels zu verdampfen. Hierbei können die flüchtigen Magnesiumverbindungen auch auf einem inerten Trägermaterial, beispielsweise Kieselsäure, Aluminiumoxid oder Kohle, aufgebracht sein. Die zu verdampfende Magnesiumverbindung kann sich ausserhalb oder innerhalb des Reaktors befinden. Vorzugsweise ist sie so angeordnet, dass die erhitzten Reaktionskomponenten zuerst durch eine die Magnesiumverbindung enthaltende Zone und dann durch die den Katalysator enthaltende Zone des Reaktors strömen. Im Prinzip können diese beiden Zonen aber auch ineinander übergehen oder gegebenenfalls miteinander vermischt sein.

Die flüchtigen Magnesiumverbindungen können kontinuierlich oder diskontinuierlich in die Reaktionszone eingebracht werden. Bei der bevorzugten kontinuierlichen Zugabe der Magnesiumverbindung beträgt sie 0,01 bis 200 ppm, vorzugsweise 0,1 bis 50 ppm, bezogen auf das Gewicht des über den Katalysator geleiteten Gasstroms. Bei diskontinuierlichem Zusatz der Magnesiumverbindung können je nach der Dauer des Zusatzes gegebenenfalls auch grössere Mengen der Magnesiumverbindung dem Gasgemisch beigemischt werden. Die zugeführte Menge lässt sich über die Temperatur und das Volumen des über die Magnesiumverbindung geleiteten Gases regeln.

Der die flüchtige Magnesiumverbindung, Kohlenmonoxid und Wasserstoff enthaltende Gasstrom wird dann an dem Rhodium, Magnesium und Halogenid enthaltenden Katalysator umgesetzt.

Bei der besonders bevorzugten Ausführungsform in einer Kreisgasapparatur, in der nach Abtrennung der kondensierbaren Reaktionsprodukte das nicht umgesetzte Gasgemisch nach Zugabe von frischem Synthesegas wieder in den Reaktor zurückgeführt wird, kann die Magnesiumverbindung entweder dem Kreisgas, dem frischen Synthesegas oder dem Gemisch der beiden zugegeben werden.

Man kann die Lebensdauer der Katalysatoren aber auch auf eine andere Art als durch Zufuhr von Magnesiumverbindungen verlängern, nämlich dadurch, dass man während der Synthesegasumsetzung der Reaktionszone zusammen mit den gasförmigen Reaktionskomponenten Halogenwasserstoff oder flüchtige organische Halogenverbindungen, die keinen Schwefel oder Stickstoff im Molekül enthalten und die unter den Reaktionsbedingungen Halogenwasserstoff abspalten, kontinuierlich oder diskontinuierlich zuführt.

Diese bevorzugte Ausführungsform des erfindungsgemässen Verfahrens bietet ebenso wie die Zufuhr von Magnesiumverbindungen den Vorteil, dass die für die Umsetzung verwendeten Katalysatoren in ihrer Aktivität und Selektivität auch nach mehr als 1000 Stunden noch nahezu unverändert sind.

Als Halogenwasserstoffe können Chlorwasserstoff, Bromwasserstoff oder Jodwasserstoff bzw. deren Gemische eingesetzt oder durch Umsetzung von Halogenen mit Wasserstoff bzw. Synthesegas im Reaktionsraum erzeugt werden. Der bevorzugte Halogenwasserstoff ist Chlorwasserstoff.

Flüchtige organische Halogenverbindungen, die keinen Schwefel oder Stickstoff im Molekül enthalten und die unter den Reaktionsbedingungen Halogenwasserstoff abspalten, sind Alkyl-, Aryl- und Aralkylhalogenide mit einem oder mehreren Halogenatomen im Molekül, wie beispielsweise Dichlormethan, Tetrachlorkohlenstoff, Ethyljodid, 1,1-Dichlorethan, Allylchlorid, tert.-Butylchlorid oder Benzylchlorid, ferner gesättigte oder ungesättigte Halogencarbonsäuren, -aldehyde, -alkohole oder -ether der aliphatischen, cycloaliphatischen oder aromatischen Reihe, zum Beispiel Mono-, Di- oder Trichloressigsäure, Jodessigsäure, Bromaceton, $\alpha,\beta$-Dichlordiethylether, 3-Chlor-crotonsäure (cis- oder trans) und p-Chlorbenzoesäure. Weiterhin sind auch Carbonsäurehalogenide geeignet, wie z.B. Acetylchlorid, -bromid und -jodid oder Mono-, Di- oder Trichloracetylchlorid, die unter Einwirkung des bei der Synthesegas-Umsetzung entstehenden Wassers sehr leicht Halogenwasserstoff abspalten können. Die bevorzugte organische Halogenverbindung ist Acetylchlorid.

Es ist nicht erforderlich, dass die Halogenwasserstoff-Abspaltung aus den flüchtigen organischen Halogenverbindungen quantitativ erfolgt, sondern es genügt bereits die Abspaltung geringer Mengen Halogenwasserstoff, um die Lebensdauer der Katalysatoren wesentlich zu verlängern.

Die Halogenwasserstoffe oder die Halogenwasserstoff abspaltenden organischen Verbindungen werden zusammen mit den gasförmigen Reaktionskomponenten in die Reaktionszone gebracht, wobei verschiedene Methoden angewendet werden können. So kann man die Halogenwasserstoffe oder die organischen Halogenverbindungen in gelöster Form, beispielsweise als Lösung in Wasser, Ethanol oder Essigsäure, in den heissen Gasstrom einbringen. Es besteht weiter die Möglichkeit, das gesamte Reaktionsgas – oder im Nebenstrom nur einen Teil dieses Gasstromes – vor Eintritt in die Reaktionszone über die feste oder flüssige organische Halogenverbindung zu leiten. Durch Wahl der Gasmenge, des Drucks und der Temperatur kann dabei entsprechend dem Partialdruck der eingesetzten Verbindung die gewünschte Menge zugegeben werden. Weiter können die organischen Halogenverbindungen auch in imprägnierter Form auf einem inerten Träger wie Kieselsäure, Aluminiumoxid oder Kohle aufgebracht sein, über den dann die Reaktionskomponenten, d.h. CO und $H_2$, geleitet werden.

Die Halogenwasserstoffe bzw. die flüchtigen organischen Halogenverbindungen können kontinuierlich oder diskontinuierlich in die Reaktionszone eingebracht werden. Bei der bevorzugten kontinuierlichen Zugabe beträgt ihre Konzentration im allgemeinen 0,01 bis 500 ppm, vorzugsweise 0,1 bis 100 ppm, bezogen auf das Gewicht des über den Katalysator geleiteten Gasstroms. Bei diskontinuierlichem Zusatz können je nach der Dauer des Zusatzes gegebenenfalls auch grössere Mengen dem Gasgemisch beigemischt werden. Die zugesetzten Mengen sind dabei umgekehrt proportional zur Dauer des Zusatzes.

Der den Halogenwasserstoff bzw. die flüchtige organische Halogenverbindung, Kohlenmonoxid und Wasserstoff enthaltende Gasstrom wird dann an dem Rhodium, Magnesium und Halogenid enthaltenden Katalysator umgesetzt.

Bei der besonders bevorzugten Ausführungsform in einer Kreisgasapparatur, in der nach Abtrennung der kondensierbaren Reaktionsprodukte das nicht umgesetzte Gasgemisch nach Zugabe von frischem Synthesegas wieder in den Reaktor zurückgeführt wird, kann der Halogenwasserstoff bzw. die organische Halogenverbindung entweder dem Kreisgas, dem frischen Synthesegas oder dem Gemisch der beiden zugegeben werden.

Die beiden beschriebenen Massnahmen zur Verlängerung der Lebensdauer der Katalysatoren – Zufuhr von Magnesiumverbindungen oder von Halogenwasserstoff bzw. organischer Halogenverbindung – können auch gemeinsam angewandt werden.

Beispiele 1–12 und Vergleichsbeispiele 1 und 2:
A) Allgemeine Versuchsbeschreibung

Die Apparatur besteht aus einem beheizten Reaktionsrohr von 1 m Länge und 16 mm innerem Durchmesser aus korrosionsbeständigem Stahl mit einer koaxial angebrachten Thermometerhülse von 6 mm äusserem Durchmesser, einem nachgeschalteten Kodensator, einer Vorlage für das Kondensat und einem Kompressor für die Rückführung eines Teils der nichtkondensierten Gase zum Reaktor (Kreisgas). Es werden jeweils 100 ml der unten beschriebenen Katalysatoren eingefüllt. Nach Spülen der Apparatur mit Stickstoff wird zunächst mit einem Synthesegas der Zusammensetzung 49 Vol.-% CO, 49 Vol.-% $H_2$, 1 Vol.-% $CO_2$, 1 Vol.-% $N_2$ (und geringe Mengen anderer Komponenten) ein Druck von 100 bar eingestellt und der Reaktor auf 275°C aufgeheizt. Während des Aufheizens und im weiteren Versuchsverlauf werden stündlich 450 Nl Synthesegas der obigen Zusammensetzung über die Saugseite des Kompressors dem Kreisgas zugeführt und zusammen mit diesem über den Katalysator geleitet. Das den Reaktor verlassende Gasgemisch wird in dem solegekühlten Kondensator auf etwa +5°C abgekühlt und die kondensierten Anteile in der Vorlage aufgefangen. Das nicht kondensierte Restgas wird nach Vermischen mit frischem Synthesegas über den Kompressor wieder dem Reaktor zugeführt. Zur Aufrechterhaltung des Drucks und zur Ausschleusung von Nebenprodukten wird ein Teil des Restgases über ein Druckventil als Abgas abgeleitet. Nach dieser Methode werden die nachstehend beschriebenen Katalysatoren geprüft. In der Tabelle sind die Laufzeit der Versuche, die Raumzeitausbeuten an sauerstoffhaltigen $C_2$-Produkten pro Liter Katalysator und Stunde bei Versuchsbeginn und -ende, die prozentuale Verteilung von Essigsäure, Acetaldehyd und Ethanol, bezogen auf den $C_2$-Anteil des Kondensats, sowie die Selektivitäten zu diesen Verbindungen (in Mol.-% CO, bezogen auf umgesetztes CO) zusammengestellt. Geringe Mengen an entstandenem Ethylacetat bzw. Acetaldehyddiethylacetal werden in Essigsäure, Ethanol bzw. Acetaldehyd umgerechnet.

Katalysatorherstellung

Die mittels ESCA (electron spectroscopy for chemical analysis) beim Rh-3d-5/2-Term ermittelte Bindungsenergie beträgt bei den frisch reduzierten und ebenso bei den gebrauchten Katalysatoren der folgenden Beispiele jeweils 308 eV. Ein Signal für Rh-Metalle war nicht vorhanden.

Beispiel 1

40 g einer Kieselsäure mit 270 m²/g BET-Oberfläche, 1,22 ml/g Porenvolumen, 0,4 kg/l Schüttgewicht, einem pH von 7,4 (gemessen an einem Granulat von 2–3 mm Durchmesser), sowie einem Gehalt von 99,35 Gew.-% $SiO_2$ und 0,2 Gew.-% Na werden mit einer Lösung von 7,5 g Magnesiumchlorid (56%ig) in 45 ml Wasser getränkt, 2 Stunden bei 70°C und 2 Stunden bei 150°C getrocknet. Anschliessend wird 30 Minuten bei 900°C gesintert. Nach dem Erkalten wird mit einer Lösung von 5,7 g $RhCl_3$ . x $H_2O$ (37,8 Gew.-% Rh) in 45 ml Wasser getränkt und in gleicher Weise wie oben getrocknet. Der Katalysator wird in einem Strömungsrohr aus Glas durch 3stündiges Überleiten von 30 Nl/h Wasserstoff bei 225–275°C unter Normaldruck reduziert. Er enthält nach der Reduktion 4,6 Gew.-% Rh, 2,3 Gew.-% Mg und 4,9 Gew.-% Cl.

Die Raumzeitausbeute beträgt zu Versuchsbeginn 470 g sauerstoffhaltige $C_2$-Verbindungen pro Liter Katalysator und Stunde. Davon entfallen 60 Gew.-% auf Essigsäure, 32,8 Gew.-% auf Acetaldehyd und 7,2 Gew.-% auf Ethanol.

Nach einer Versuchszeit von 620 Stunden beträgt der Cl-Gehalt noch 3,8 Gew.-%.

Vergleichsbeispiel 1 (mit Mg- und Halogenfreiem Katalysator)

Es werden 6,1 g $Rh(NO_3)_3$ · 2 $H_2O$ (31,8 Gew.-% Rh) in 45 ml Wasser gelöst und auf 40 g des in Beispiel 1 genannten Kieselsäureträgers aufgezogen. Nach einer Ruhezeit von 2 Stunden wird der Katalysator bei 80°C und 260 mbar unter Überleiten von 1 Nl/h Stickstoff getrocknet. Der Katalysator wird, wie in Beispiel 1 angegeben, reduziert und enthält 4,6 Gew.-% Rh.

Vergleichsbeispiel 2 (mit Halogen-freiem Katalysator)

10,6 g $Mg(NO_3)_2$ · 6 $H_2O$ werden in 43 ml Wasser gelöst, auf 40 g des in Beispiel 1 genannten Kieselsäureträgers aufgezogen, bei 120°C getrocknet und anschliessend 30 Minuten bei 800°C gesintert. Nach dem Erkalten wird mit einer Lösung von 6,3 g $Rh(NO_3)_3$ · 2 $H_2O$ (31,8 Gew.-% Rh) in 45 ml Wasser imprägniert, bei 80°C im Vakuum von 260 mbar unter 1 Nl/h Stickstoff getrocknet und wie in Beispiel 1 reduziert. Der Katalysator enthält nach der Reduktion 4,6 Gew.-% Rh und 2,3 Gew.-% Mg.

Beispiel 2

Als Träger verwendet man ein natürliches, handelsübliches Magnesiumsilikat der folgenden Zusammensetzung: 56–60 Gew.-% $SiO_2$, 1,2–3,5 Gew.-% $Al_2O_3$, 0,5–1,3 Gew.-% $Fe_2O_3$, 22,0–26,3 Gew.-% MgO, 5,5–8,0 Gew.-% Summe $CO_2$, Alkalioxide, CaO und $TiO_2$. Der Glühverlust beträgt 10 Gew.-%, das spez. Gewicht 2,45–2,6 g/ml und die Korngrösse 2–3 mm.

54 g (etwa 0,1 l) des oben beschriebenen Trägers werden mit einer Lösung von 7,0 g $RhCl_3$ · x $H_2O$ (37,8 Gew.-% Rh) in 45 ml Wasser getränkt und bei 150°C getrocknet. Man reduziert den Katalysator wie in Beispiel 1 beschrieben, jedoch mit 60 Nl/h eines Gemisches von Stickstoff und Wasserstoff im Vol.-Verhältnis 1:1, anstelle von 30 Nl/h Wasserstoff. Der reduzierte Katalysator enthält 4,6 Gew.-% Rh, 13,5 Gew.-% Mg und 2,3 Gew.-% Cl. Nach 450 Stunden Versuchszeit beträgt der Cl-Gehalt noch 2,1 Gew.-%.

Beispiel 3 (Reduktion im Reaktor)

54 g (etwa 0,1 l) des in Beispiel 2 beschriebenen Trägers werden mit einer Lösung von 7,0 g $RhCl_3$ · x $H_2O$ (37,8 Gew.-% Rh) in 45 ml Wasser getränkt und bei 150°C getrocknet. 100 ml dieses Katalysators werden unreduziert in den Reaktor gegeben, unter einem Stickstoffstrom von 30 Nl/h drucklos bis auf 225°C aufgeheizt und bei dieser Temperatur durch Überleiten von 60 Nl/h eines Gemisches von Kohlenmonoxid und Wasserstoff im Volumenverhältnis 1:1 reduziert. In Abweichung von der allgemeinen Versuchsbeschreibung wird mit dem Synthesegas bei 225°C ein Druck von 100 bar eingestellt und dann der Katalysator auf die Reaktionstemperatur von 275°C aufgeheizt. Der Katalysator enthält nach der Reduktion 4,6 Gew.-% Rh, 13,5 Gew.-% Mg und 2,25 Gew.-% Cl. Der Cl-Gehalt beträgt nach 420 Stunden Versuchsdauer noch 2,0 Gew.-%.

Beispiel 4

Es wird der in Beispiel 2 beschriebene natürliche Magnesiumsilikatträger gewaschen und getrocknet. Er hat danach folgende Zusammensetzung: 65,5 Gew.-% $SiO_2$, 3,6 Gew.-% $Al_2O_3$, 0,5 Gew.-% $Fe_2O_3$ und 14,0 Gew.-% MgO. Das Schüttgewicht beträgt 537 g/l, das Porenvolumen 0,99 ml/g.

54 g dieses Trägers werden mit einer Lösung von 7,0 g $RhCl_3$ · x $H_2O$ (37,8 Gew.-% Rh) in 49 ml Wasser getränkt und bei 150°C getrocknet. Der Katalysator wird nach der in Beispiel 1 beschriebenen Methode reduziert. Er enthält 4,6 Gew.-% Rh, 7,8 Gew.-% Mg und 2,9 Gew.-% Cl.

Beispiel 5

54 g des nach Beispiel 4 hergestellten Magnesiumsilikat-Trägers werden mit einer Lösung von 14,4 g $RhBr_3$· 2 $H_2O$ (27,2 Gew.-% Rh) in 49 ml Wasser getränkt und getrocknet. Die Reduktionsmethode ist die gleiche wie in Beispiel 1. Der reduzierte Katalysator enthält 6,0 Gew.-% Rh, 7,3 Gew.-% Mg und 7,3 Gew.-% Br. Im Laufe der 310stündigen Versuchszeit nimmt der Br-Gehalt nur auf 6,6 Gew.-% ab.

**Beispiel 6**

Es werden 10 g $RhCl_3 \cdot x\ H_2O$ (37,8 Gew.-% Rh) in 20 ml Wasser gelöst, mit einer Lösung von 18,2 g Kaliumjodid in 20 ml Wasser kalt vermischt und sofort nach dem Vermischen auf 54 g Magnesiumsilikat-Träger der in Beispiel 4 angegebenen Zusammensetzung aufgezogen.

Der imprägnierte Katalysator wird 48 Stunden bei Raumtemperatur gelagert und anschliessend bei 80 °C im Vakuum unter Stickstoff getrocknet. Er enthält nach dem Trocknen 4,6 Gew.-% Rh, 5,5 Gew.-% Mg, 5,2 Gew.-% K, 16,9 Gew.-% J und 4,7 Gew.-% Cl.

Die Reduktion wird, wie in Beispiel 1 beschrieben, durch 3stündiges Überleiten von 30 Nl/h Wasserstoff bei 225–275 °C unter Normaldruck durchgeführt. Nach der Reduktion enthält der Katalysator 4,7 Gew.-% Cl und 5,6 Gew.-% J.

**Beispiel 7**

40 g des in Beispiel 1 beschriebenen Trägers werden mit einer Lösung von 4,4 g $[Rh\ (CO)_2Cl]_2$ (Rh-Gehalt 52,94 Gew.-%) und 6,8 g wasserfreiem Magnesiumacetat in 43 ml Methanol getränkt und bei 80 °C getrocknet. Der Katalysator, der 4,6 Gew.-% Rh, 2,3 Gew.-% Mg und 1,9 Gew.-% Cl enthält, wird ohne reduktive Vorbehandlung in den Reaktor gegeben.

**Beispiel 8**

40 g des in Beispiel 1 beschriebenen Trägers werden mit einer Lösung von 9,1 g $Mg_3\ [Rh\ Cl_6]_2 \cdot 7\ H_2O$ (24,8 Gew.-% Rh, 8,8 Gew.-% Mg) in 45 ml Methanol getränkt, bei 80 °C getrocknet und, wie in Beispiel 1 beschrieben, reduziert. Nach der Reduktion enthält der Katalysator 4,6 Gew.-% Rh, 1,6 Gew.-% Mg und 4,0 Gew.-% Cl.

**Beispiel 9** (Katalysatorherstellung mit Gefriertrocknung)

Es werden 4,9 g $Mg\ [Rh^I\ (CO)_2Cl_2]_2$ (42,5 Gew.-% Rh) unter Stickstoff bei 0 °C in 46 ml Methanol gelöst und auf 40 g eines gekühlten Kieselsäureträgers (Zusammensetzung wie in Beispiel 1) aufgezogen, auf –10 °C abgekühlt und bei 13 mbar bis zum konstanten Gewicht getrocknet. Der fertige Katalysator enthält 4,6 Gew.-% Rh, 0,55 Gew.-% Mg und 3,2 Gew.-% Cl. 100 ml des Katalysators werden ohne weitere reduktive Behandlung in den Reaktor gefüllt.

**Beispiel 10**

Man verfährt wie in Beispiel 1, tränkt den $MgCl_2$-haltigen, gesinterten Träger aber nach dem Erkalten mit einer Lösung von 2,4 g $Rh\ Cl_3 \cdot x\ H_2O$ (37,8 Gew.-% Rh) in 45 ml Wasser. Nach dem Trocknen wird der Katalysator in der in Beispiel 1 beschriebenen Weise reduziert. Er enthält nach der Reduktion 2,0 Gew.-% Rh, 2,3 Gew.-% Mg und 3,8 Gew.-% Cl.

**Beispiel 11**

Man verfährt wie in Beispiel 1, verwendet aber für die Imprägnierung mit $RhCl_3$ eine Lösung von 10,2 g $RhCl_3 \cdot x\ H_2O$ (37,8 Gew.-% Rh) in 45 ml Wasser. Die weitere Verarbeitung ist die gleiche wie in Beispiel 1. Der reduzierte Katalysator enthält 8,0 Gew.-% Rh, 2,25 Gew.-% Mg und 5,4 Gew.-% Cl.

**Beispiel 12**

40 g des in Beispiel 1 beschriebenen Kieselsäureträgers werden mit einer Lösung von 7,5 g (56%ig) und 5,0 g $RhCl_3 \cdot xH_2O$ (37,8 Gew.-% Rh) in 45 ml Wasser getränkt und 2 Stunden bei 70 °C und 2 Stunden bei 150 °C getrocknet. Der Katalysator wird nach der in Beispiel 1 beschriebenen Methode reduziert. Er enthält nach der Reduktion 4,0 Gew.-% Rh, 2,3 Gew.-% Mg und 4,3 Gew.-% Cl.

**C) Versuchsergebnisse**

Die Ergebnisse sind in der folgenden Tabelle dargestellt.

Tabelle

Reaktionsbedingungen: 100 bar, 275 °C, Einsatzgas 450 Nl/h $CO:H_2$ = 1:1, Katalysatorvolumen 0,1 l, AcOH = Essigsäure, AcH = Acetaldehyd, EtOH = Ethanol

| Beispiel | Katalysatorzusammensetzung | | | Versuchsdauer in Stunden | Raumzeitausbeute an sauerstoffhaltigen $C_2$-Verb. in g/l·h | | Zusammensetzung der $C_2$-Verbindungen in Gew.-% | | | Selektivität Mol% CO umgesetzt zu | |
|---|---|---|---|---|---|---|---|---|---|---|---|
| | Rh [Gew.-%] | Mg [Gew.-%] | Träger | | Versuchsbeginn | Versuchsende | AcOH | AcH | EtOH | $C_2$-Verb. | $CH_4$ |
| 1 | 4,6 | 2,3 | $SiO_2$ | 620 | 470 | 445 | 60,0 | 32,8 | 7,2 | 85,0 | 7,1 |
| Vergl. bsp. 1 | 4,6 | – | $SiO_2$ | 180 | 52 | 33 | 42,3 | 11,5 | 46,1 | 48,0 | 30,0 |
| Vergl. bsp. 2 | 4,6 | 2,3 | $SiO_2$ | 265 | 275 | 183 | 62,9 | 20,3 | 16,7 | 61,0 | 33,0 |
| 2 | 4,6 | 13,5 | $MgSiO_3$ | 450 | 415 | 338 | 19,2 | 12,7 | 63,1 | 78,0 | 17,5 |
| 3 | 4,6 | 13,5 | $MgSiO_3$ | 420 | 436 | 418 | 25,6 | 17,0 | 57,3 | 82,0 | 9,9 |
| 4 | 4,6 | 7,8 | $MgSiO_3$ | 580 | 495 | 462 | 29,6 | 12,5 | 57,8 | 90,5 | 5,9 |

## Tabelle (Fortsetzung)

Reaktionsbedingungen: 100 bar, 275°C, Einsatzgas 450 Nl/h $CO:H_2$ = 1:1, Katalysatorvolumen 0,1 l, AcOH = Essigsäure, AcH = Acetaldehyd, EtOH = Ethanol

| Beispiel | Katalysator-zusammensetzung | | | Ver-suchs-dauer in Stun-den | Raumzeit-ausbeute an sauerstoffhal-tigen $C_2$-Verb. in g/l·h | | Zusammensetzung der $C_2$-Verbindungen in Gew.-% | | | Selektivität Mol% CO umgesetzt zu | |
|---|---|---|---|---|---|---|---|---|---|---|---|
| | Rh [Gew.-%] | Mg [Gew.-%] | Träger | | Ver-suchs-beginn | Ver-suchs-ende | AcOH | AcH | EtOH | $C_2$-Verb. | $CH_4$ |
| 5 | 6,0 | 7,3 | $MgSiO_3$ | 310 | 522 | 486 | 39,8 | 20,7 | 39,4 | 77,2 | 12,7 |
| 6 | 4,6 | 7,7 | $MgSiO_3$ | 215 | 422 | 387 | 53,1 | 17,1 | 29,8 | 82,5 | 10,9 |
| 7 | 4,6 | 2,3 | $SiO_2$ | 380 | 465 | 452 | 66,7 | 25,2 | 8,1 | 83,0 | 10,5 |
| 8 | 4,6 | 1,6 | $SiO_2$ | 250 | 480 | 453 | 56,7 | 26,7 | 16,6 | 86,0 | 9,8 |
| 9 | 4,6 | 0,55 | $SiO_2$ | 220 | 495 | 475 | 53,3 | 27,2 | 19,3 | 84,5 | 9,4 |
| 10 | 2,0 | 2,3 | $SiO_2$ | 350 | 370 | 352 | 62,1 | 28,7 | 9,2 | 78,5 | 13,0 |
| 11 | 8,0 | 2,3 | $SiO_2$ | 350 | 532 | 490 | 53,7 | 30,5 | 15,8 | 86,0 | 8,2 |
| 12 | 4,0 | 2,3 | $SiO_2$ | 280 | 410 | 375 | 50,7 | 35,1 | 14,2 | 78,2 | 12,8 |

### Beispiel 13

100 g einer Kieselsäure mit 270 m²/g BET-Oberfläche, 1,27 ml/g Porenvolumen, 0,4 kg/l Schüttgewicht, einem pH von 7,4 (als Granulat von 2–3 mm Durchmesser) sowie einem Gehalt von 99,35 Gew.-% $SiO_2$ und 0,2 Gew.-% Na werden mit einer Lösung von 18,75 g Magnesiumchlorid (56%ig) in 112 ml Wasser getränkt und dann 2 Stunden bei 70°C und 2 Stunden bei 150°C getrocknet. Anschliessend wird 30 Minuten bei 900°C gesintert. Nach dem Erkalten wird die Kieselsäure mit einer Lösung von 14,25 g $RhCl_3 \cdot H_2O$ (37,8 Gew.-% Rh) in 112 ml Wasser getränkt und in gleicher Weise wie oben getrocknet. Der Katalysator wird in einem Strömungsrohr aus Glas durch 3stündiges Überleiten von 75 Nl/h Wasserstoff bei 225 bis 275°C unter Normaldruck reduziert. Er enthält nach der Reduktion 4,6 Gew.-% Rh, 2,3 Gew.-% Mg und 4,9 Gew.-% Cl.

100 ml des Katalysators werden in ein als Reaktor dienendes senkrecht stehendes Strömungsrohr aus korrosionsbeständigem Stahl von 16 mm innerem Durchmesser und 1 m Länge gegeben, das mit äusserer Salzbadheizung, Temperaturmessvorrichtung, nachgeschaltetem Kühler, Vorlage für das Kondensat und Entspannungsvorrichtung versehen ist. Nach Spülen mit Stickstoff werden bei 120 bar und 280°C stündlich 235 Nl eines Gasgemisches, das 49 Vol.% Kohlenmonoxid, 49 Vol.% Wasserstoff, 1 Vol.% $CO_2$ und geringe Mengen Stickstoff enthält, über den Katalysator geleitet. In das heisse Gasgemisch werden in einem vor dem Reaktor angebrachten, ebenfalls auf 280°C beheizten Vorheizer stündlich 10 ml einer wässrigen 0,07 gewichtsprozentigen Magnesiumacetat-Lösung eingespritzt. Die Reaktionsgase werden nach Verlassen des Reaktors auf etwa +5°C abgekühlt und die nicht kondensierbaren Anteile entspannt. Es fallen als Kondensat stündlich etwa 27 g Essigsäure, 13 g Acetaldehyd und 4 g Ethanol in Form einer wässrigen Lösung an, entsprechend einer Raumzeitausbeute von 440 g an diesen sauerstoffhaltigen $C_2$-Produkten pro Liter Katalysator und Stunde. Geringe Mengen (etwa 2 Gew.-%, bezogen auf die genannten $C_2$-Produkte) an entstandenem Ethylacetat bzw. Acetaldehyddiethylacetal wurden in Essigsäure, Ethanol bzw. Acetaldehyd umgerechnet und sind in den angegebenen Werten enthalten. Dies gilt auch für die folgenden Beispiele.

Der CO-Umsatz beträgt 39,5 Vol.%, die Selektivität zu den sauerstofhaltigen $C_2$-Produkten 82%, bezogen auf umgesetztes Kohlenmonoxid. Nach 1400 Stunden sind die Raumzeitausbeute, der CO-Umsatz und die Selektivität noch unverändert.

### Vergleichsbeispiel 3:

Man verfährt wie in Beispiel 13, gibt jedoch anstelle der wässrigen Magnesiumacetatlösung stündlich 10 ml destilliertes Wasser in den Vorheizer. Die Raumzeitausbeute beträgt nach 200 Stunden gleichfalls 440 g sauerstoffhaltige $C_2$-Produkte, nach 550 Stunden noch 425 g, nach 1000 Stunden nur noch 320 g, jeweils pro Liter Katalysator und Stunde. Die prozentuale Zusammensetzung der $C_2$-Produkte ist die gleiche wie in Beispiel 13. Der CO-Umsatz geht in dieser Zeit von 39,5% auf 34,6% und die Selektivität zu den sauerstoffhaltigen $C_2$-Produkten von 82% auf 68% zurück.

### Beispiel 14

In den Reaktor der in Beispiel 13 beschriebenen Apparatur werden 100 ml des dort genannten Katalysators gefüllt. Die Versuchsausführung ist die gleiche wie in Beispiel 13, es entfällt jedoch das Einspritzen der Magnesiumacetatlösung in den Vorheizer. Stattdessen werden in den Vorheizer 100 ml Kieselsäure gegeben, die mit einer Lösung von 20 g Magnesiumchlorid (56%ig) in 35 g Wasser getränkt und anschliessend getrocknet wurden. Die Reaktionskomponenten Kohlenmonoxid und Wasserstoff werden nun in derselben Zusammensetzung wie in Beispiel 13 über den Vorheizer in den Reaktor geleitet. Unter den in Beispiel 13 genannten Reaktionsbedingungen werden stündlich 30,5 g Essigsäure, 10,5 g Acetaldehyd und 3,8 g Ethanol als wässriges Kondensat erhalten. Der CO-Umsatz beträgt 38,7%, die Selektivität zu den sauerstoffhaltigen $C_2$-Produkten 83,4% bezogen auf umgesetztes Kohlenmonoxid. Die Raumzeitausbeute, der CO-Umsatz und die Selektivität sind auch nach mehr als 120 Stunden noch unverändert.

Beispiel 15

Als Träger verwendet man ein natürlich vorkommendes, handelsübliches Magnesiumsilikat, das nach dem Waschen und Trocknen folgende Zusammensetzung hat: 65,5 Gew.-% $SiO_2$, 3,6 Gew.-% $Al_2O_3$, 0,5 Gew.-% $Fe_2O_3$ und 14,0 Gew.-% MgO. Das Schüttgewicht beträgt 537 g/l und das Porenvolumen 0,99 ml/g.

108 g dieses Trägers (200 ml) werden mit einer Lösung von 12,6 g $RhCl_3 \cdot$ x $H_2O$ (37,8 Gew.-% Rh) in 98 ml Wasser getränkt und bei 150°C getrocknet. Der Katalysator wird in einem Strömungsrohr aus Glas durch 3stündiges Überleiten von 75 Nl/h Stickstoff bei 225 bis 275°C unter Normaldruck reduziert. Er enthält nach der Reduktion 4,2 Gew.-% Rh, 7,9 Gew.-% Mg und 3,8 Gew.-% Cl.

100 ml des reduzierten Katalysators werden in den in Beispiel 14 beschriebenen Reaktor gegeben, der zusätzlich mit einem Kompressor für die Kreisgasführung eines Teils des Restgasgemisches versehen ist. Der Vorheizer wird mit 100 ml eines Kieselsäureträgers gefüllt, der vorher mit einer Lösung von 15 g Magnesiumacetat in 40 g Wasser getränkt und anschliessend getrocknet wurde.

Nach Spülen mit Stickstoff wird zunächst mit einem Synthesegas (49 Vol.% CO, 49 Vol.% $H_2$, 1 Vol.% $CO_2$, Spuren $N_2$) ein Druck von 120 bar eingestellt und der Katalysator auf 280°C aufgeheizt. Während des Aufheizens und im weiteren Versuchsverlauf werden stündlich 300 Nl des Synthesegases über die Saugseite des Kompressors dem Kreisgas zugeführt und zusammen mit dem Kreisgas erst durch den auf 280°C beheizten Vorheizer und anschliessend durch den Reaktor geleitet. Das den Reaktor verlassende Gasgemisch wird in einem solegekühlten Kondensator auf ca. +5°C abgekühlt, und die kondensierten Anteile werden in der Vorlage aufgefangen. Das nicht kondensierte Restgas wird nach Mischung mit Frischgas über den Kompressor wieder dem Vorheizer und dem Reaktor zugeführt. Zur Konstanthaltung des Drucks und zur Ausschleusung von Nebenprodukten wird ein Teil des Restgases über ein Druckhalteventil als Abgas abgeleitet.

Es fallen stündlich 49 g sauerstoffhaltige $C_2$-Verbindungen (15 g Essigsäure, 6 g Acetaldehyd und 28 g Ethanol) in Form einer wässrigen Lösung an, entsprechend einer Raumzeitausbeute von 490 g/lh. Der CO-Umsatz beträgt durchschnittlich 35% vom Einsatz und die Selektivität zu den sauerstoffhaltigen $C_2$-Produkten 86,6%, bezogen auf umgesetztes Kohlenmonoxid. Raumzeitausbeute, CO-Umsatz und Selektivität sind nach 120 Stunden noch unverändert.

Vergleichsbeispiel 4:

Man verfährt wie im Beispiel 15, füllt aber den Vorheizer mit reiner, nicht getränkter Kieselsäure. Unter sonst gleichen Versuchsbedingungen wie im Beispiel 15 und unter Verwendung von 100 ml Katalysator der dort genannten Zusammensetzung liegt die Raumzeitausbeute in den ersten 380 Stunden bei 475 g sauerstoffhaltigen $C_2$-Produkten pro Liter Katalysator und Stunde, nach insgesamt 720 Stunden bei 435 g/lh und nach 1200 Stunden noch bei 360 g/lh, bei gleicher prozentualer Zusammensetzung des Produktgemisches wie im Beispiel 15. Der CO-Umsatz fällt in der genannten Zeit von 35% auf 28,6% und die Selektivität zu den sauerstoffhaltigen $C_2$-Produkten von 86% auf 77,9%, bezogen auf umgesetztes Kohlenmonoxid.

Beispiel 16

100 g einer Kieselsäure mit 270 m²/g BET-Oberfläche, 1,27 ml/g Porenvolumen, 0,4 kg/l Schüttgewicht, einem pH von 7,4 (als Granulat von 2–3 mm Durchmesser), sowie einem Gehalt von 99,35 Gew.-% $SiO_2$ und 0,2 Gew.-% Na werden mit einer Lösung von 14,7 g Magnesiumchlorid (56%ig) in 112 ml Wasser getränkt und dann 2 Stunden bei 70°C sowie 2 Stunden bei 150°C getrocknet. Anschliessend wird 30 Minuten bei 800°C gesintert. Nach dem Erkalten wird die Kieselsäure mit einer Lösung von 14,0 g $RhCl_3 \cdot$ x $H_2O$ (38,0 Gew.-% Rh) in 112 ml Wasser getränkt und in gleicher Weise wie oben getrocknet. Der Katalysator wird in einem Strömungsrohr aus Glas durch 3stündiges Überleiten von 75 Nl/h Wasserstoff bei 225 bis 275°C unter Normaldruck reduziert. Er enthält nach der Reduktion 4,5 Gew.-% Rh, 1,8 Gew.-% Mg und 4,7 Gew.-% Cl.

100 ml des Katalysators werden in ein als Reaktor dienendes senkrecht stehendes Strömungsrohr aus korrosionsbeständigem Stahl von 16 mm innerem Durchmesser und 1 m Länge gegeben, das mit äusserer Salzbadheizung, Temperaturmessvorrichtung, nachgeschaltetem Kühler, Vorlage für das Kondensat und Entspannungsvorrichtung versehen ist.

Nach Spülen mit Stickstoff werden bei 100 bar und 290°C stündlich 320 Nl eines Gasgemisches, das 49 Vol.% Kohlenmonoxid, 49 Vol.% Wasserstoff, 1 Vol.% $CO_2$ und geringe Mengen Stickstoff enthält, über den Katalysator geleitet. In das heisse Gasgemisch werden in einem vor dem Reaktor angebrachten, ebenfalls auf 290°C beheizten Vorheizer stündlich 10 ml einer wässrigen 0,1 gewichtsprozentigen Salzsäure eingespritzt.

Die Reaktionsgase werden nach Verlassen des Reaktors auf etwa +5°C abgekühlt und die nicht kondensierten Anteile entspannt. Es fallen als Kondensat stündlich etwa 34 g Essigsäure, 8 g Acetaldehyd und 4,5 g Ethanol in Form einer wässrigen Lösung an, entsprechend einer Raumzeitausbeute von 465 g an diesen sauerstoffhaltigen $C_2$-Produkten pro Liter Katalysator und Stunde. Geringe Mengen (etwa 2 Gew.-%, bezogen auf die genannten $C_2$-Produkte) an entstandenem Ethylacetat bzw. Acetaldehyddiethylacetal wurden in Essigsäure, Ethanol bzw. Acetaldehyd umgerechnet und sind in den angegebenen Werten enthalten. Dies gilt auch für die folgenden Beispiele.

Der CO-Umsatz beträgt 30 Mol.%, die Selektivität zu den sauerstoffhaltigen $C_2$-Produkten 80,6%, bezogen auf umgesetztes Kohlenmonoxid. Nach 1450 Stunden beträgt die Raumzeitausbeute noch 450 g pro Liter Katalysator und Stunde. Die Selektivität ist unverändert.

Vergleichsbeispiel 5:

Man verfährt wie in Beispiel 16, gibt jedoch anstelle der verdünnten Salzsäure stündlich 10 ml destilliertes Wasser in den Vorheizer. Die Raumzeitausbeute beträgt nach 200 Stunden gleichfalls 465 g sauerstoffhaltige $C_2$-Produkte, nach 750 Stunden noch 410 g und nach 1200 Stunden nur noch 370 g, jeweils pro Liter Katalysator und Stunde. Die prozentuale Zusammensetzung der $C_2$-Produkte ist die gleiche wie in Beispiel 16. Der CO-Umsatz geht in dieser Zeit von 30% auf 27,5% und die Selektivität zu den sauerstoffhaltigen $C_2$-Produkten von 80,6 auf 69,6% zurück.

Beispiel 17

Als Träger verwendet man ein natürlich vorkommendes, handelsübliches Magnesiumsilikat, das nach dem Waschen und Trocknen folgende Zusammensetzung hat: 65,5 Gew.-% $SiO_2$, 3,6 Gew.-% $Al_2O$, 0,5 Gew.-% $Fe_2O_3$ und 14,0 Gew.-% MgO. Das Schüttgewicht beträgt 537 g/l und das Porenvolumen 0,99 ml/g.

108 g dieses Trägers (200 ml) werden mit einer Lösung von 12,6 g $RhCl_3 \cdot x H_2O$ (37,8 Gew.-% Rh) in 98 ml Wasser getränkt und bei 150 °C getrocknet. Der Katalysator wird in einem Strömungsrohr aus Glas durch 3stündiges Überleiten von 75 Nl/h Stickstoff bei 225 bis 275 °C unter Normaldruck reduziert. Er enthält nach der Reduktion 4,1 Gew.-% Rh, 7,5 Gew.-% Mg und 3,6 Gew.-% Cl.

100 ml des reduzierten Katalysators werden in den in Beispiel 16 beschriebenen Reaktor gegeben, der zusätzlich mit einem Kompressor für die Kreisgasführung eines Teils des Restgasgemisches versehen ist.

Nach Spülen mit Stickstoff wird zunächst mit einem Synthesegas (49 Vol.% CO, 49 Vol.% $H_2$, 1 Vol.% $CO_2$, Spuren $N_2$) ein Druck von 100 bar eingestellt und der Katalysator auf 290 °C aufgeheizt. Während des Aufheizens und im weiteren Versuchsverlauf werden stündlich 350 Nl des Synthesegases über die Saugseite des Kompressors dem Kreisgas zugeführt und zusammen mit dem Kreisgas erst durch den auf 290 °C beheizten Vorheizer und anschliessend durch den Reaktor geleitet. In den Vorheizer werden stündlich 18 ml einer 0,3 gewichtsprozentigen Lösung von Methylenchlorid in Methanol gegeben. Das den Reaktor verlassende Gasgemisch wird in einem solegekühlten Kondensator auf ca. +5 °C abgekühlt, und die kondensierten Anteile werden in der Vorlage aufgefangen. Das nicht kondensierte Restgas wird nach Mischung mit Frischgas über den Kompressor wieder dem Vorheizer und dem Reaktor zugeführt. Zur Konstanthaltung des Drucks und zur Ausschleusung von Nebenprodukten wird ein Teil des Restgases über ein Druckhalteventil als Abgas abgeleitet.

Es fallen stündlich 48 g sauerstoffhaltige $C_2$-Verbindungen (14 g Essigsäure, 5 g Acetaldehyd und 29 g Ethanol) in Form einer wässrigen Lösung an, entsprechend einer Raumzeitausbeute von 480 g/l·h. Der CO-Umsatz beträgt durchschnittlich 32% vom Einsatz und die Selektivität zu den sauerstoffhaltigen $C_2$-Produkten 79,6%, bezogen auf umgesetztes Kohlenmonoxid. Raumzeitausbeute, CO-Umsatz und Selektivität sind nach 1750 Stunden noch unverändert.

Vergleichsbeispiel 6:

Man verfährt wie im Beispiel 17, dosiert aber in den Vorheizer stündlich 18 ml Methanol. Unter sonst gleichen Versuchsbedingungen wie im Beispiel 17 und unter Verwendung von 100 ml Katalysator der dort genannten Zusammensetzung liegt die Raumzeitausbeute in den ersten 400 Stunden bei 470 g sauerstoffhaltigen $C_2$-Produkten pro Liter Katalysator und Stunde, nach insgesamt 800 Stunden bei 460 g/l·h und nach 1250 Stunden noch bei 425 g/l·h bei gleicher prozentualer Zusammensetzung des Produktgemisches wie im Beispiel 17. Der CO-Umsatz fällt in der genannten Zeit von 31% auf 29,5% und die Selektivität zu den sauerstoffhaltigen $C_2$-Produkten von 80,5% auf 76,5%, bezogen auf umgesetztes Kohlenmonoxid.

Beispiel 18

Man verfährt wie in Beispiel 17, gibt aber in den Vorheizer anstelle der methanolischen Methylenchlorid-Lösung stündlich 25 ml Diethylether. Die Raumzeitausbeute zu den sauerstoffhaltigen $C_2$-Produkten fällt unter diesen Bedingungen von 490 g/l·h nach 100 Stunden bis auf 450 g/l·h nach 800 Stunden ab. Im weiteren Versuchsverlauf werden dann in den Vorheizer 25 ml einer 0,2 gewichtsprozentigen Lösung von Acetylchlorid in Diethylether gegeben. Die Raumzeitausbeute bleibt jetzt praktisch konstant und beträgt nach insgesamt 1800 Stunden Versuchszeit noch 440–445 g pro Liter Katalysator und Stunde.

Patentansprüche

1. Verfahren zur Herstellung von Essigsäure, Ethanol, Acetaldehyd und gegebenenfalls ihren Folgeprodukten durch Umsetzung von Kohlenmonoxid mit Wasserstoff in Gegenwart von Rhodium, dadurch gekennzeichnet, dass man einen Katalysator verwendet, der auf einem Träger Salze oder Komplexverbindungen des Rhodiums in einer Wertigkeitsstufe unter drei, Halogenidionen und als Cokatalysator Magnesium in Form von Salzen oder Komplexverbindungen enthält.

2. Verfahren nach Anspruch 1, dadurch gekennzeichnet, dass man als Träger Kieselsäure oder Silikate verwendet.

3. Verfahren nach Anspruch 1 oder 2, dadurch gekennzeichnet, dass man als Halogenidionen Chloride verwendet.

4. Verfahren nach Anspruch 1 bis 3, dadurch gekennzeichnet, dass man Rhodium, Magnesium und Halogenid in Form der Komplexverbindung $Mg_3[RhCl_6]_2$ oder $Mg[Rh(CO)_2Cl_2]_2$ einsetzt.

5. Verfahren nach Anspruch 1 bis 4, dadurch gekennzeichnet, dass man die Salze oder Komplexverbindungen des Rhodiums in einer Wertigkeitsstufe unter drei durch Reduktion von entsprechenden geträgerten Verbindungen des dreiwertigen Rhodiums unterhalb 300 °C erzeugt.

6. Verfahren nach Anspruch 1 bis 5, dadurch gekennzeichnet, dass man der Reaktionszone Magnesiumsalze oder -verbindungen, die unter den Reaktionsbedingungen verdampfbar sind, während der Umsetzung zusammen mit den gasförmigen Einsatzkomponenten, kontinuierlich oder diskontinuierlich zuführt.

7. Verfahren nach Anspruch 6, dadurch gekennzeichnet, dass man Magnesiumacetat oder Magnesiumchlorid verwendet.

8. Verfahren nach Anspruch 1 bis 7, dadurch gekennzeichnet, dass man der Reaktionszone Halogenwasserstoff oder flüchtige organische Halogenverbindungen, die keinen Schwefel oder Stickstoff im Molekül enthalten und die unter den Reaktionsbedingungen Halogenwasserstoff abspalten, während der Umsetzung zusammen mit den gasförmigen Einsatzkomponenten kontinuierlich oder diskontinuierlich zuführt.

9. Verfahren nach Anspruch 8, dadurch gekennzeichnet, dass man als Halogenwasserstoff Chlorwasserstoff verwendet.

10. Verfahren nach Anspruch 8, dadurch gekennzeichnet, dass man als organische Halogenverbindung Acetylchlorid verwendet.

## Claims

1. A process for the manufacture of acetic acid, ethanol, acetaldehyde and possibly secondary products thereof by reaction of carbon monoxide with hydrogen in the presence of rhodium, characterized by using a catalyst containing, applied onto a carrier, salts or complex compounds of rhodium in a valency stage below 3, halide ions and as cocatalyst magnesium in the form of salts or complex compounds.

2. The process as claimed in claim 1, characterized by using as carrier silicic acid or silicates.

3. The process as claimed in claim 1 or 2, characterized by using as halide ions chlorides.

4. The process as claimed in claims 1 to 3, characterized by using rhodium, magnesium and halide in the form of the complex compounds $Mg_3[RhCl_6]_2$ or $Mg[Rh(CO)_2Cl_2]_2$.

5. The process as claimed in claims 1 to 4, characterized by preparing the salts or complex compounds of rhodium in a valency stage below 3 by reduction of corresponding supported compounds of trivalent rhodium at a temperature below 30 °C.

6. The process as claimed in claims 1 to 5, characterized by feeding continuously or discontinuously to the reaction zone during the reaction, magnesium salts or magnesium compounds vaporizable under the reaction conditions, in conjunction with the gaseous feed components.

7. The process as claimed in claim 6, characterized by using magnesium acetate or magnesium chloride.

8. The process as claimed in claims 1 to 7, characterized by feeding continuously or discontinuously to the reaction zone hydrogen halide or volatile organic halogen compounds containing no sulfur or nitrogen in the molecule and splitting off hydrogen halide under the reaction conditions in conjunction with the gaseous feed components.

9. The process as claimed in claim 8, characterizedf by using as hydrogen halide hydrogen chloride.

10. The process as claimed in claim 8, characterized by using as organic halogen compound acetylchloride.

## Revendications

1. Procédé de préparation d'acide acétique, d'éthanol, d'acétaldéhyde et éventuellement de leurs produits successeurs, par réaction du monoxyde de carbone avec l'hydrogène en présence de rhodium, procédé caractérisé en ce qu'on utilise un catalyseur qui contient, sur un support, des sels ou des complexes du rhodium à une valence inférieure à 3, des ions halogénures, et, comme co-catalyseur, du magnésium sous la forme de sels ou de complexes.

2. Procédé selon la revendication 1, caractérisé en ce qu'on utilise, comme supports, de la silice ou des silicates.

3. Procédé selon l'une des revendications 1 et 2, caractérisé en ce qu'on utilise des chlorures comme ions halogénures.

4. Procédé selon l'une quelconque des revendications 1 à 3, caractérisé en ce qu'on utilise le rhodium, le magnésium et l'halogénure sous la forme du complexe $Mg_3[RhCl_6]_2$ ou $Mg[Rh(CO)_2Cl_2]_2$.

5. Procédé selon l'une quelconque des revendications 1 à 4, caractérisé en ce que les sels ou complexes du rhodium à une valence inférieure à 3 sont produits par réduction de composés correspondants du rhodiumt trivalent, déposés sur support, à une température inférieure à 300 °C.

6. Procédé selon l'une quelconque des revendications 1 à 5, caractérisé en ce qu'on introduit dans la zone réactionnelle, en continu ou en discontinu, au cours de la réaction, en même temps que les composantes gazeuses à mettre en jeu, des sels ou composés du magnésium susceptibles d'être évaporés dans les conditions de la réaction.

7. Procédé selon la revendication 6, caractérisé en ce qu'on utilise de l'acétate de magnésium ou du chlorure de magnésium.

8. Procédé selon l'une quelconque des revendictions 1 à 7, caractérisé en ce qu'on introduit dans la zone réactionnelle, en continu ou en discontinu, au cours de la réaction, en même temps que les composantes gazeuses à mettre en jeu, un halogénure d'hydrogène ou des composés organiques halogénés volatils ne contenant ni soufre ni azote dans leur molécule et capables de libérer un halogénure d'hydrogène dans les conditions de la réaction.

9. Procédé selon la revendication 8, caractérisé en ce qu'on utilise le chlorure d'hydrogène comme halogénure d'hydrogène.

10. Procédé selon la revendication 8, caractérisé en ce qu'on utilise le chlorure d'acétyle comme composé organique halogéné.